# EUROPEAN PATENT APPLICATION

(11) **EP 4 741 382 A1**
(43) Date of publication of application: **13.05.2026**
(21) Application number: 24835990.3
(22) Date of filing: 28.06.2024
(51) Int. Cl.: C07D 285/08

(54) **NOVEL METHOD FOR PRODUCING 3-METHYL-1,2,4-THIADIAZOLE-5-CARBOHYDRAZIDE**

(30) Priority: 03.07.2023 JP 2023109329
(71) Applicant: AGC Inc., Chiyoda-ku Tokyo 100-8405 (JP)
(72) Inventor: SUZUKI, Hideo, Tokyo 100-8405 (JP); YAMAZAKI, Yusuke, Tokyo 100-8405 (JP); KODERA, Ikumi, Tokyo 100-8405 (JP); SHIMIZU, Yukiyo, Tokyo 100-8405 (JP)
(74) Representative: Müller-Boré & Partner Patentanwälte PartG mbB
(86) International application number: PCT/JP2024/023470
(87) International publication number: WO 2025/009477

(57) **Abstract**

The present invention aims to provide a method for producing 3-methyl-1,2,4-thiadiazole-5-carbohydrazide safely and in high yield. The present invention relates to a method for producing 3-methyl-1,2,4-thiadiazole-5-carboxamide or a salt thereof without using toxic carbon monoxide gas. According to the present invention, a safe and high-yielding production method of 3-methyl-1,2,4-thiadiazole-5-carbohydrazide, which is an important synthetic intermediate for fezolinetant useful as a medicament for the treatment of sex hormone-dependent diseases, can be provided.

## Description

### [Technical Field]

The present invention relates to a novel, safe, and practical method for producing 3-methyl-1,2,4-thiadiazole-5-carbohydrazide which is an important synthetic intermediate for fezolinetant which was developed as a selective antagonist of the neurokinin (NK)-3 receptor, and is particularly useful as a compound for the treatment of sex hormone-dependent diseases.

### [Background Art]

Fezolinetant (generic name) (VEOZAH (registered trademark)) was developed as a selective antagonist of NK-3 receptor, and is particularly useful as a compound for the treatment and/or prophylaxis of sex hormone-dependent diseases (e.g., moderate to severe vasomotor symptoms associated with menopause, such as facial flushing, hot flashes, and the like). Fezolinetant is (R)-(4-fluorophenyl)-(8-methyl-3-(3-methyl-1,2,4-thiadiazol-5-yl)-5,6-dihydro-[1,2,4]triazolo[4,3-a]pyrazin-7(8H)-yl)methanone and described in Patent Literature 1.

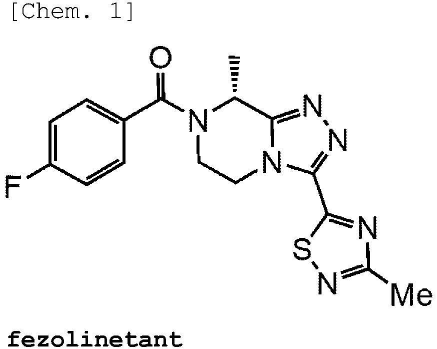

Deuterated fezolinetant, (R)-(4-fluorophenyl)-(8-methyl-3-(3-(methyl-d3)-1,2,4-thiadiazol-5-yl)-5,6-dihydro-[1,2,4]triazolo[4,3-a]pyrazin-7(8H)-yl)methanone was also developed for the same purpose and is described in Patent Literature 2.

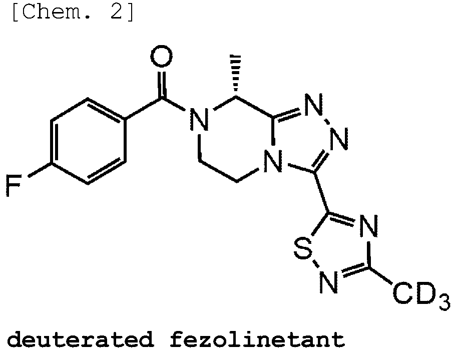

Patent Literatures 1 and 2 disclose, as a synthesis method of fezolinetant and deuterated fezolinetant, a synthesis method going through 3-methyl-1,2,4-thiadiazole-5-carbohydrazide or a deuterated form thereof, which is a compound represented by the formula (I): 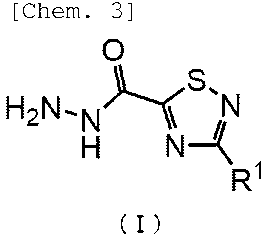 wherein R¹ is methyl or methyl-d₃, as an important intermediate.

Patent Literature 3 discloses a synthesis method of a non-deuterated intermediate, 3-methyl-1,2,4-thiadiazole-5-carbohydrazide (2n): 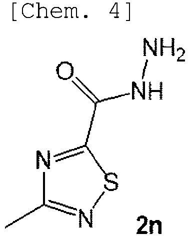 and in the synthesis method, it is prepared from the corresponding methyl ester (a compound represented by the following formula (2.2n)), which in turn is prepared in a single step from acetamide (a compound represented by the following formula (2.0n)), chlorocarbonylsulfenyl chloride (a compound represented by the following formula (2.0n')), and methyl cyanoformate (a compound represented by the following formula (2.0n")).

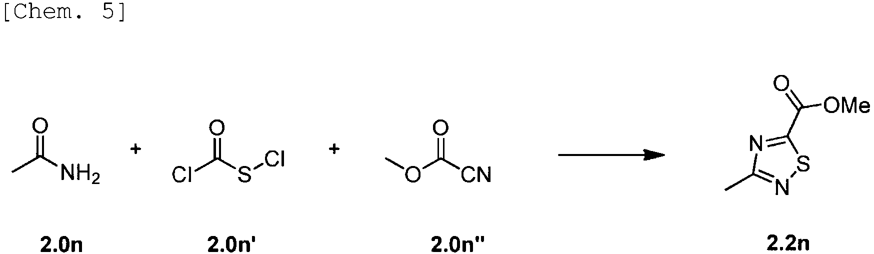

However, chlorocarbonylsulfenyl chloride and methyl cyanoformate are hazardous reagents that pose problems in large-scale procurement and are difficult to scale up. This synthetic route also results in sulfur impurities that adversely affect the quality of the final pharmaceutical product. Even after optimization, the overall yield of 3-methyl-1,2,4-thiadiazole-5-carbohydrazide remains below 30%. The synthesis of the corresponding deuterated intermediate, 3-(methyl-d₃)-1,2,4-thiadiazole-5-carbohydrazide, also has similar drawbacks (Patent Literature 2).

Patent Literature 4 discloses, as a synthesis method of an intermediate compound represented by the following formula (I-1): 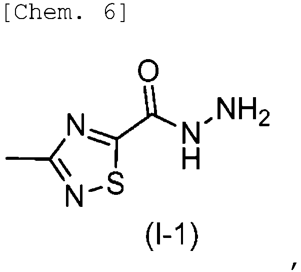
a synthesis method including alkoxycarbonylation by lithium exchange reaction or by carbon monoxide insertion reaction in the presence of a metal catalyst of the corresponding halogenated intermediate, or a compound represented by the following formula (III-1-a): 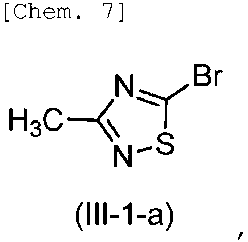
or a compound represented by the following formula (III-1-b): 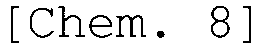 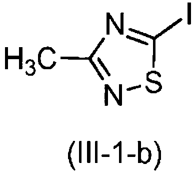
to obtain the corresponding ester form, a compound represented by the following formula (II-1-b): 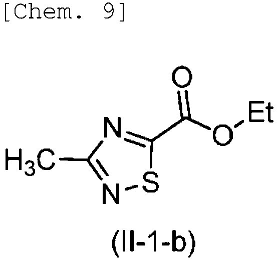
or a salt thereof, a synthesis method via which is disclosed. However, the yield of the alkoxycarbonylation reaction by lithium exchange is as low as 29%. Furthermore, although the alkoxycarbonylation reaction via carbon monoxide insertion in the presence of a metal catalyst affords a compound represented by formula (II-1-b) in good yield, it requires the use of toxic carbon monoxide gas and is limited in terms of scale-up, thus leaving issues for practical application.

Therefore, the development of a safe, high-yielding, scalable, and practical method for producing 3-methyl-1,2,4-thiadiazole-5-carbohydrazide which is an important synthetic intermediate for fezolinetant is desired.

### [Citation List]

### [Patent Literature]

[Patent Literature 1]
   WO 2014/154895
[Patent Literature 2]
   WO 2019/012033
[Patent Literature 3]
   WO 2013/050424
[Patent Literature 4]
   WO 2020/128003

### [Summary of Invention]

### [Technical Problem]

An object of the present invention is to provide a safe, high-yielding, and practical method for producing a compound represented by the following formula (I): 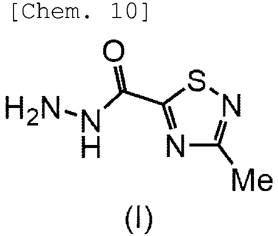 or a salt thereof which is an important synthetic intermediate for fezolinetant.

### [Solution to Problem]

The present inventors have conducted intensive studies in an attempt to solve the above-mentioned problem and found that a compound represented by the formula (3): 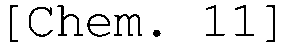 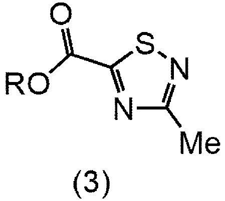
wherein R is a C₁₋₄ alkyl group,
or a salt thereof can be produced in a high yield without using carbon monoxide gas, by using a compound represented by the formula (2): 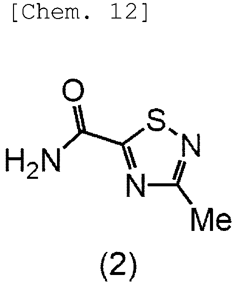
or a salt thereof which is obtained by a step of reacting a compound represented by the formula (1): 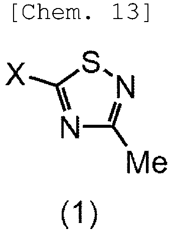
wherein X is a halogen atom,
or a salt thereof with an alkali metal cyanide,
and that, as a result, a safe and practical production method of a compound represented by the formula (I): 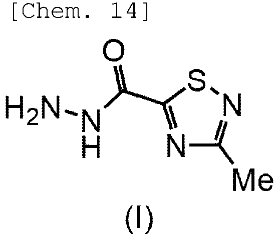
or a salt thereof, which is an important synthetic intermediate for fezolinetant, can be provided, which resulted in the completion of the present invention.

That is, the present invention is as follows.
[1] A method for producing a compound represented by the formula (2): 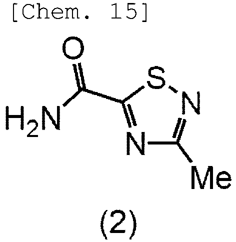
   or a salt thereof,
   comprising a step of reacting a compound represented by the formula (1): 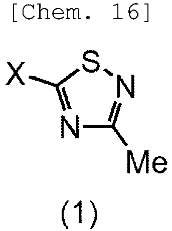
   wherein X is a halogen atom, or a salt thereof
   with an alkali metal cyanide in a mixed solvent of acetonitrile and water in the presence of an organic base.
[2] The production method of the above-mentioned [1], wherein
   X is a chlorine atom or a bromine atom.
[3] The production method of the above-mentioned [1], wherein
   the organic base is triethylamine, N,N-diisopropylethylamine, N-methylmorpholine, pyridine, 2,6-dimethylpyridine, N,N-dimethyl-4-aminopyridine, imidazole, 1,8-diazabicyclo[5.4.0]undec-7-ene, 1,4-diazabicyclo[2.2.2]octane, or 1,5-diazabicyclo[4.3.0]non-5-ene.
[4] The production method of the above-mentioned [1], wherein
   the alkali metal cyanide is potassium cyanide or sodium cyanide.
[5] The production method of the above-mentioned [1], wherein
   the mixed solvent of acetonitrile and water has a mixing ratio (v/v) in the range of 1 to 10 as water/acetonitrile.
[6] A method for producing a compound represented by the formula (3): 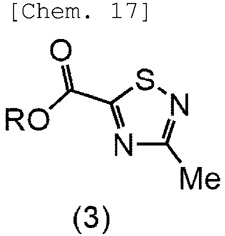
   wherein R is a C₁₋₄ alkyl group, or a salt thereof,
      comprising a step of reacting a compound represented by the formula (2) which is obtained by the production method of any of the above-mentioned [1] to [5], or a salt thereof with thionyl chloride in the presence of alcohol represented by the following formula:

      [Chem. 18] ROH
   wherein R is as defined above.
[7] The production method of the above-mentioned [6], wherein
   the alcohol is methanol or ethanol.
[8] A method for producing a compound represented by the formula (I): 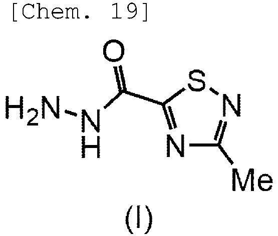 or a salt thereof,
   comprising a step of reacting the compound represented by the formula (3) which is obtained by the production method of the above-mentioned [6] or [7] or a salt thereof with hydrazine or a hydrate thereof in a solvent.

### [Advantageous Effects of Invention]

According to the present invention, 3-methyl-1,2,4-thiadiazole-5-carbohydrazide represented by the aforementioned formula (I) or a salt thereof, which is an important synthetic intermediate for fezolinetant, can be produced in good yield without using toxic carbon monoxide gas. Therefore, a practical production method of fezolinetant can be provided which is safer than conventional methods and can be scaled up.

### [Description of Embodiments]

The definitions of the terms used in the present specification are described below.

In the present specification, a compound represented by a formula may be referred to by adding the formula number to the "compound". For example, a compound represented by the formula (1) is referred to as "compound (1)".

In the present specification, a numerical range shown by "~" or "-" means a numerical range with the numbers before and after "~" or "-" as the lower limit or upper limit.

In the present specification, when the element symbol "C" is used with a numerical range indicated by numbers before and after "-" attached to the name of any group, it represents any group having an integer number of carbon atoms, with the numbers before and after "-" as the lower limit and upper limit. For example, an alkyl group having 1 to 4 carbon atoms is sometimes denoted as a "C₁₋₄ alkyl group" which represents each of -CH₃, -C₂H₅, -C₃H₇, -C₄H₉, and the like.

In the present specification, the "halogen atom" means a fluorine atom, a chlorine atom, a bromine atom, or an iodine atom.

In the present specification, the "C₁₋₄ alkyl group" means a linear or branched chain saturated hydrocarbon group having 1 to 4 carbon atoms, and examples of the C₁₋₄ alkyl group include methyl, ethyl, propyl, isopropyl, butyl, isobutyl, sec-butyl, and tert-butyl.

In the present specification, examples of the "alkali metal cyanide" include lithium cyanide, potassium cyanide, sodium cyanide, cesium cyanide, and the like.

In the present specification, the "organic base" is an organic compound that acts as a base. The organic base is generally a proton acceptor containing a nitrogen atom that can be easily protonated, and examples thereof include amine, nitrogen-containing heterocyclic compounds, and the like. The "organic base" is not particularly limited and examples thereof include triethylamine (TEA), N,N-diisopropylethylamine (DIPEA), N-methylmorpholine (NMO), N,N-dimethyl-4-aminopyridine (DMAP), imidazole, 1,8-diazabicyclo[5.4.0]undec-7-ene (DBU), 1,4-diazabicyclo[2.2.2]octane (DABCO), 1,5-diazabicyclo[4.3.0]non-5-ene (DBN), and the like.

In the present specification, the "prophylaxis" includes preventing the onset of a disease, delaying the onset of a disease, and preventing the development of a pathology in a subject.

In the present specification, the "treatment" includes curing a disease, ameliorating the pathology of a disease (e.g., one or more symptoms), and inhibiting the progression (of the severity) of a disease in a subject.

In the present specification, the "subject" refers to a subject to which a medicament (pharmaceutical composition) containing an effective amount of an active ingredient necessary for the prophylaxis and/or treatment of a disease or a pathological condition of a disease is administered. The "subject" may be a human or a non-human animal, particularly a mammal (e.g., mouse, rat, guinea pig, hamster, rabbit, cat, dog, cow, sheep, monkey, etc.).

In the present specification, "a salt thereof" is not particularly limited as long as it is a salt formed by reacting the target compound with a base or an acid. Since the present invention relates to a method for producing an important synthetic intermediate for fezolinetant, which is useful as a medicament, a salt that can be used as a medicament (a pharmaceutically acceptable salt) is preferred. When each compound used in the production method of the present invention forms a basic salt or an acidic salt by reacting with a base or an acid, it refers to such salt.

Examples of the "basic salt" include sodium salt, potassium salt, lithium salt, magnesium salt, calcium salt, aluminum salt, ammonium salt, tetramethylammonium salt, N-methylmorpholine salt, diethylamine salt, triethylamine salt, tributylamine salt, diisopropylethylamine salt, dicyclohexylamine salt, N-methylpiperidine salt, pyridine salt, 4-pyrrolidinopyridine salt, picoline salt, choline salt, diolamine salt, meglumine salt, olamine salt, tromethamine salt, 2-(diethylamino)ethanol salt, 4-(2-hydroxyethyl)morpholine salt, arginine salt, glycine salt, lysine salt, benzathine salt and the like.

Examples of the "acidic salt" include hydrofluoride, hydrochloride, hydrobromide, hydroiodide, nitrate, perchlorate, sulfate, bisulfate, borate, camsylate, cyclamate, carbonate, bicarbonate, phosphate, hexafluorophosphate, formate, lactate, trifluoroacetate, mesylate, trifluoromethanesulfonate, esilate, edisilate, benzoate, besylate, tosylate, acetate, malate, fumarate, succinate, malonate, citrate, ascorbate, tartrate, oxalate, maleate, adipate, nicotinate, aspartate, gluconate, glucuronate, pyroglutamate, stearate, palmitate, tannate, isethionate, naphthoate, orotate, pamoate, xinafoate, gluceptate, and hibenzate.

In the present invention, a compound represented by the formula (I): 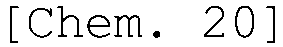 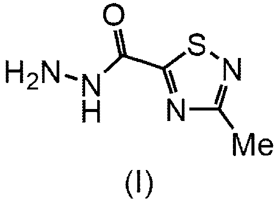 (compound (I)) or a salt thereof also includes a compound in which the 3-position methyl group is deuterated, i.e., 3-(methyl-d3)-1,2,4-thiadiazole-5-carbohydrazide or a salt thereof.

### (Production method of the present invention)

The production method of the present invention is described in the following.

A compound represented by the formula (I): 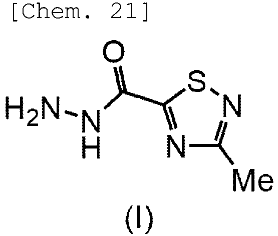 (compound (I)) or a salt thereof can be produced by the following production method, the below-mentioned Example, a method analogous thereto, and the like.

Each raw material compound may form a salt as long as it does not inhibit the reaction, and examples of such salts include the same salts as those described above.

Unless a specific production method is described, the starting compounds can be easily obtained from those on the market and used, or can be produced by a method known per se or a method analogous thereto. In addition, intermediates produced in the following production methods may be isolated and purified by a method such as column chromatography, recrystallization, or distillation, or may be used in the next step without isolation.

In the present specification, the contents of all patent literature, non patent literature, or references expressly cited are hereby incorporated by reference in their entirety.

In the present invention, compound (I) or a salt thereof is produced by a step of reacting a compound represented by the formula (1): 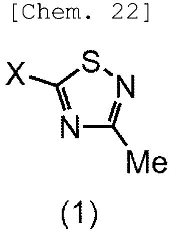
wherein X is a halogen atom (compound (1)) or a salt thereof and an alkali metal cyanide (MCN) in a mixed solvent of acetonitrile and water in the presence of an organic base (step 1) to produce a compound represented by the formula (2): 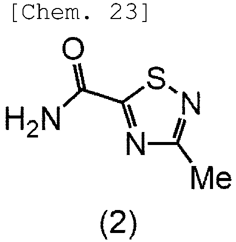
(compound (2)) or a salt thereof, a step of converting the compound to a compound represented by the formula (3): 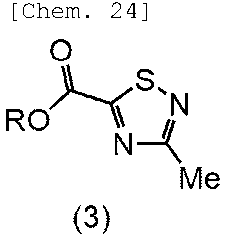
wherein R is a C₁₋₄ alkyl group (compound (3)) or a salt thereof (step 2), and then a step of reacting the compound with hydrazine or a hydrate thereof (step 3).

Each step in the production method of the present invention is described in detail in the following.

### (Step 1)

In this step, compound (1) or a salt thereof is reacted with an alkali metal cyanide (MCN) in a mixed solvent of acetonitrile and water in the presence of an organic base to produce compound (2) or a salt thereof. 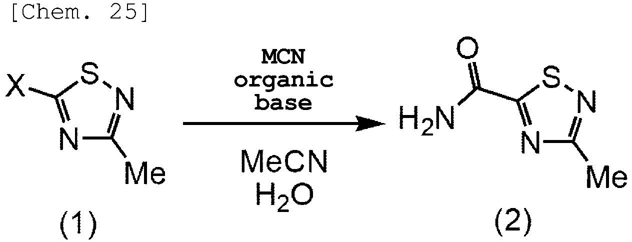 wherein X is a halogen atom, and M is an alkali metal.

Compound (1) or a salt thereof, which is synthesized by a method known per se (see, for example, WO 2020/128003 (Patent Literature 4), Chem. Ber., 1957, 90, 182-187, etc.) or a method analogous thereto, can be suitably used. Compound (1) or a salt thereof is preferably 5-chloro-3-methyl-1,2,4-thiadiazole or 5-bromo-3-methyl-1,2,4-thiadiazole, more preferably 5-chloro-3-methyl-1,2,4-thiadiazole.

Alkali metal cyanide (MCN) is not particularly limited, and examples thereof include lithium cyanide, potassium cyanide, sodium cyanide, cesium cyanide, and the like, and potassium cyanide or sodium cyanide is preferred, and sodium cyanide is more preferred.

The amount of the alkali metal cyanide to be used is 1 to 3 moles, preferably 1.1 to 1.5 moles, per 1 mole of compound (1) or a salt thereof.

The organic base is not particularly limited and examples thereof include triethylamine (TEA), N,N-diisopropylethylamine (DIPEA), N-methylmorpholine (NMO), pyridine, 2,6-dimethylpyridine, N,N-dimethyl-4-aminopyridine (DMAP), imidazole, 1,8-diazabicyclo[5.4.0]undec-7-ene (DBU), 1,4-diazabicyclo[2.2.2]octane (DABCO), 1,5-diazabicyclo[4.3.0]non-5-ene (DBN), and the like, and N,N-diisopropylethylamine (DIPEA), N,N-dimethyl-4-aminopyridine (DMAP), 1,8-diazabicyclo[5.4.0]undec-7-ene (DBU), or 1,4-diazabicyclo[2.2.2]octane (DABCO) is preferred, and 1,4-diazabicyclo[2.2.2]octane (DABCO) is more preferred.

The amount of the organic base to be used is generally 0.01 to 2 moles, preferably 0.05 to 0.2 moles, per 1 mole of compound (1) or a salt thereof.

This reaction can be performed in a mixed solvent of acetonitrile and water.

As the mixing ratio (v/v) of the mixed solvent of acetonitrile and water, water/acetonitrile is generally in the range of 0.1 to 20, preferably 1 to 10, more preferably 2 to 8.

The reaction temperature is generally 0°C to 80°C, preferably 5°C to 40°C, and the reaction time is generally 1 to 12 hr.

### (Step 2)

In this step, compound (3) or a salt thereof is produced by reacting compound (2) obtained in the aforementioned step 1 or a salt thereof with thionyl chloride in the presence of alcohol represented by the following formula:

[Chem. 26] ROH

wherein R is a C₁₋₄ alkyl group. 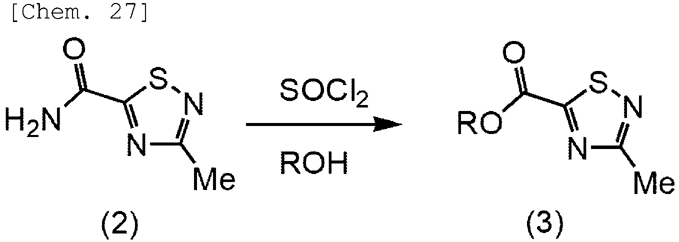 wherein R is as defined above.

The alcohol is not particularly limited as long as it has 1 to 4 carbon atoms, and is preferably methanol or ethanol.

The amount of alcohol to be used is not particularly limited and the amount of the solvent can be used.

The amount of thionyl chloride to be used is generally 1 to 3 moles, preferably 1.1 to 1.5 moles, per 1 mole of compound (2) or a salt thereof.

The reaction temperature is generally 20°C to 80°C, preferably 40°C to 70°C (heating reflux temperature), and the reaction time is generally 4 to 12 hr.

### (Step 3)

In this step, compound (3) or a salt thereof obtained in the aforementioned step 2 is reacted with hydrazine or a hydrate thereof in a solvent to produce compound (I) or a salt thereof.

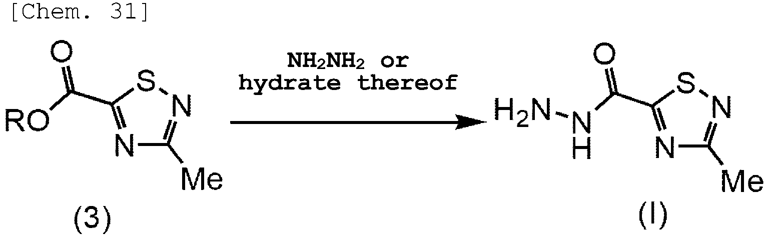

The hydrazine or a hydrate thereof is not particularly limited and hydrazine monohydrate is preferred.

The amount of the hydrazine or a hydrate thereof to be used is generally 1 to 2 moles, preferably 1 to 1.2 mole, per 1 mole of compound (3) or a salt thereof.

This reaction can be performed in a solvent that does not affect the reaction. The reaction solvent is not particularly limited, and examples thereof include aromatic hydrocarbons such as toluene, xylene, and the like; amides such as N,N-dimethylformamide, N,N-dimethylacetamide, and the like; alcohols such as methanol, ethanol, isopropanol, and the like; nitriles such as acetonitrile and the like, or a mixture thereof. Among these, a mixed solvent of alcohol and aromatic hydrocarbon is preferred, and a mixed solvent of isopropanol-toluene is more preferred.

The reaction temperature is generally -10°C to 30°C, preferably 0°C to 10°C, and the reaction time is generally 3 to 24 hr.

After completion of the reaction, compound (I) or a salt thereof can be isolated and/or purified from the reaction mixture according to a conventional method using a separation means such as concentration, crystallization, recrystallization, distillation, solvent extraction, fractionation, chromatography and the like.

Compound (I) or a salt thereof obtained in the aforementioned step 3 can be converted to fezolinetant by a method known per se (see, for example, WO 2014/154895 (Patent Literature 1), WO 2019/012033 (Patent Literature 2)), or a method analogous thereto.

The production method of the present invention does not use any carbon monoxide gas, the use of which on an industrial scale is restricted, and therefore has the advantage of being able to synthesize compound (I) or a salt thereof in good yield by a safe and simple operation compared to conventional methods.

### [Example]

The present invention is specifically explained in detail in the following by referring to Reference Examples and Examples; however, the present invention is not limited to these Examples.

In the present specification, % means mol/mol% for yield and wt% for others unless particularly indicated. The room temperature refers to a temperature of from 15 to 30°C unless particularly indicated.

Nuclear magnetic resonance spectra (NMR) were measured using ECZ400S FT-NMR manufactured by JEOL Ltd. ¹H-NMR was measured at 400 MHz using tetramethylsilane as the reference. When a deuterated solvent was used as the measurement solvent, the name of the deuterated solvent is indicated.

High-performance liquid chromatograph (HPLC) used was 1220 Infinity LC manufactured by Agilent Technologies, and the column used was Atlantis T3 (3 µm (particle size), 4.6×150 mm, manufactured by Waters).

In addition, the abbreviations used in the following Examples have the following meanings.

s: singlet
br: broad
CDCl₃: deuterochloroform
DMSO-d₆: deuterodimethyl sulfoxide
CH₂Cl₂: dichloromethane
NaOH: sodium hydroxide
NaCN: sodium cyanide
DABCO: 1,4-diazabicyclo[2.2.2]octane
MeCN: acetonitrile
MeOH: methanol
iPrOH: isopropanol

### [Reference Example 1]

### Production of 5-chloro-3-methyl-1,2,4-thiadiazole (1-1)

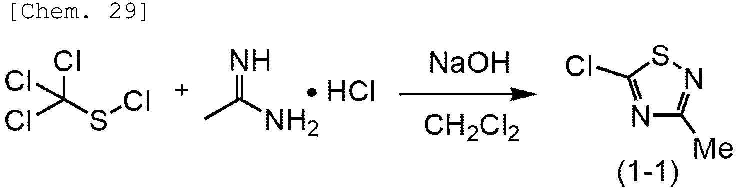

Under a nitrogen atmosphere (normal pressure) at 0°C, to acetamidine hydrochloride (100 g, 1.06 mol), trichloromethanesulfenyl chloride (196.8 g, 1.06 mol), and 6 M aqueous sodium hydroxide solution (902.7 g, 4.4 eq.) was added dichloromethane (530 mL (5.3 v/w)), and the reaction mixture was stirred at 25°C for 2 hr. Thereafter, the reaction mixture was partitioned, and the obtained organic layer was washed successively with 6 M hydrochloric acid (350 mL (3.5 v/w)) and water (350 g (3.5 v/w)). The obtained organic layer was subjected to simple distillation (vacuum pressure: 400-15 Torr, internal temperature: 25 to 60°C) to obtain the title compound (1-1) in 54% yield (quantitative by LC).
¹H-NMR (400 MHz, CDCl₃) δ 2.64 (s,3H).

### [Example 1]

### Production of 3-methyl-1,2,4-thiadiazole-5-carboxamide (2)

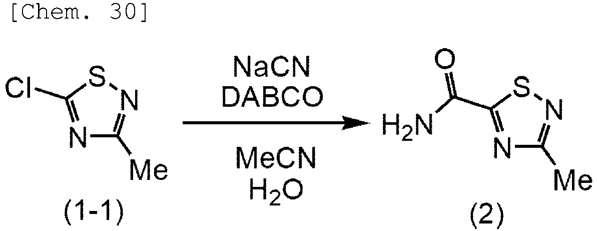

Under a nitrogen atmosphere (normal pressure) at 25°C, compound (1-1) (150 g, 1.10 mmol) obtained in Reference Example 1, acetonitrile (150 mL (1.0 v/w)), water (562.5 mL (3.75 v/w)), DABCO (12.1 g, 0.11 mmol), and sodium cyanide (68.9 g, 1.41 mmol) were mixed, and the reaction mixture was stirred at 25 to 40°C for 2 hr. Thereafter, the mixture was cooled to 5°C, filtered under reduced pressure, and the residue was washed three times with water (300 g (2 v/w)). The residue was dried under reduced pressure to obtain the title compound (2) in 67% yield (quantitative by LC).
¹H-NMR (400 MHz, CDCL₃) δ 2.72 (s,3H), 7.01 (s,2H).

### [Example 2]

### Production of methyl 3-methyl-1,2,4-thiadiazole-5-carboxylate (3-1)

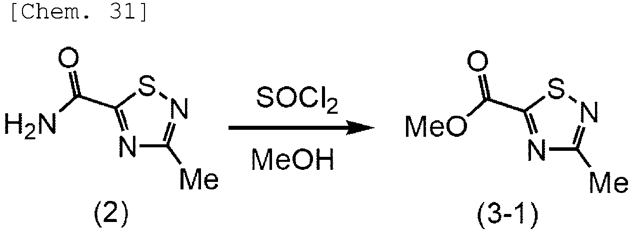

Under a nitrogen atmosphere (normal pressure) at 25°C, compound (2) (30.0 g, 0.21 mol) obtained in Example 1 and methanol (120 mL (4 v/w)) were mixed and heated to 40 to 50°C. A solution of thionyl chloride (29.5 g, 0.25 mol) in methanol (60 mL (2 v/w)), separately prepared at 0 to 20°C, was added dropwise and the mixture was stirred at 64°C for 6 hr. The methanol in the reaction mixture was removed by evaporation under reduced pressure, and the solvent was replaced with toluene. Impurities were then removed by filtration under reduced pressure, and the filtrate was separated by adding 10% aqueous sodium chloride solution (6 v/w). The obtained organic layer was concentrated under reduced pressure to 5 v/w to obtain a toluene solution of the title compound (3-1) in 82% yield (quantitative by LC).
¹H-NMR (400 MHz, CDCL₃) δ 2.79 (s,3H), 4.05 (s,3H).

### [Example 3]

### Production of 3-methyl-1,2,4-thiadiazole-5-carbohydrazide (I)

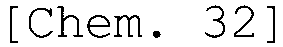

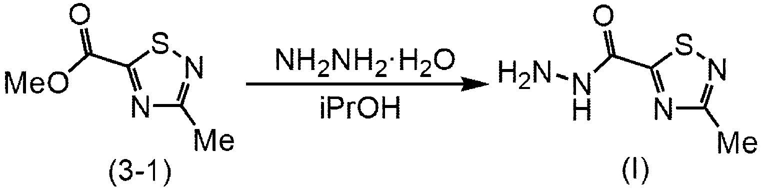

Under a nitrogen atmosphere, hydrazine monohydrate (33.2 g, 0.66 mol) was added to isopropanol (1000 mL (10 v/w)), and the mixture was cooled to 0 to 10°C. A toluene solution of compound (3-1) (0.63 mol) obtained in Example 2 was added dropwise thereto, and the mixture was stirred at 0 to 10°C for 12 hr. Thereafter, the reaction mixture was filtered under reduced pressure and the residue was washed with isopropanol (400 mL (4 v/w)) and dried under reduced pressure to obtain the title compound (I) in 95% yield (quantitative by LC).
¹H-NMR (400 MHz, DMSO-d₆) δ 2.56 (s,3H), 4.82 (br,2H), 10.56 (br,1H).

### [Industrial Applicability]

According to the present invention, 3-methyl-1,2,4-thiadiazole-5-carbohydrazide (compound (1)) or a salt thereof, which is an important synthetic intermediate for fezolinetant, can be produced in good yield without using toxic carbon monoxide gas. Therefore, a practical production method of fezolinetant can be provided which is safer than conventional methods and can be scaled up.

This application is based on a patent application No. 2023-109329 filed in Japan (filing date: July 3, 2023), the contents of which are incorporated in full herein.

## Claims

1. A method for producing a compound represented by the formula (2): 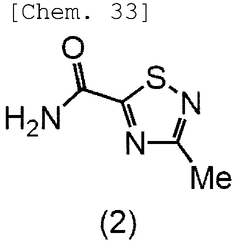
or a salt thereof,
comprising a step of reacting a compound represented by the formula (1): 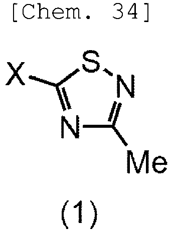
wherein X is a halogen atom, or a salt thereof
with an alkali metal cyanide in a mixed solvent of acetonitrile and water in the presence of an organic base.

2. The production method according to claim 1, wherein
X is a chlorine atom or a bromine atom.

3. The production method according to claim 1, wherein
the organic base is triethylamine, N,N-diisopropylethylamine, N-methylmorpholine, pyridine, 2,6-dimethylpyridine, N,N-dimethyl-4-aminopyridine, imidazole, 1,8-diazabicyclo[5.4.0]undec-7-ene, 1,4-diazabicyclo[2.2.2]octane, or 1,5-diazabicyclo[4.3.0]non-5-ene.

4. The production method according to claim 1, wherein
the alkali metal cyanide is potassium cyanide or sodium cyanide.

5. The production method according to claim 1, wherein
the mixed solvent of acetonitrile and water has a mixing ratio (v/v) in the range of 1 to 10 as water/acetonitrile.

6. A method for producing a compound represented by the formula (3): 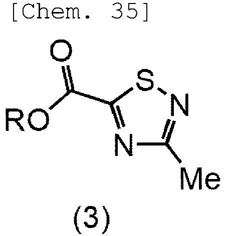
wherein R is a C₁₋₄ alkyl group, or a salt thereof,
comprising a step of reacting a compound represented by the formula (2) which is obtained by the production method according to any one of claims 1 to 5, or a salt thereof with thionyl chloride in the presence of alcohol represented by the following formula:
[Chem. 36] ROH
wherein R is as defined above.

7. The production method according to claim 6, wherein
the alcohol is methanol or ethanol.

8. A method for producing a compound represented by the formula (I): 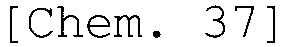 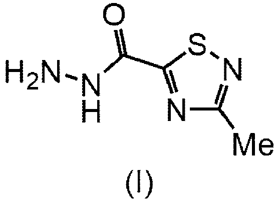 or a salt thereof,
comprising a step of reacting the compound represented by the formula (3) which is obtained by the production method according to claim 6 or a salt thereof with hydrazine or a hydrate thereof in a solvent.
